# EUROPÄISCHE PATENTSCHRIFT

(11) **EP 1 296 662 B1**
(45) Veröffentlichungstag und Bekanntmachung des Hinweises auf die Patenterteilung: **07.04.2004**
(21) Anmeldenummer: 01960466.9
(22) Anmeldetag: 05.07.2001
(51) Int. Cl.: A61K 9/70, A61K 31/19, A61K 31/195

(54) **DERMALES THERAPEUTISCHES SYSTEM, ENTHALTEND 2-(3-BENZOPHENYL)-PROPIONSÄURE ODER [O-(2,6-DICHLORANILINO)-PHENYL]-ESSIGSÄURE**
DERMAL THERAPEUTIC SYSTEM CONTAINING 2-(3-BENZOPHENYL)-PROPIONIC ACID OR '0-(2,6-DICHLORANILINO)-PHENYL!-ETHANOIC ACID
SYSTEME THERAPEUTIQUE DERMIQUE CONTENANT DE L'ACIDE 2-(3-BENZOPHENYL)-PROPIONIQUE OU DE L'ACIDE '0-(2,6-DICHLORANILINO)-PHENYL!-ACETIQUE

(30) Priorität: 05.07.2000 DE 10032537
(43) Veröffentlichungstag der Anmeldung: 02.04.2003
(73) Patentinhaber: LABTEC GESELLSCHAFT FüR TECHNOLOGISCHE FORSCHUNG UND ENTWICKLUNG MBH, 40764 Langenfeld (DE)
(72) Erfinder: CORDES, Günter, 40764 Langenfeld (DE); VOLLMER, Ulrike, 40764 Langenfeld (DE)
(74) Vertreter: Hansmann, Dierk, Dipl.-Ing.
(86) Internationale Anmeldenummer: PCT/EP2001/007712
(87) Internationale Veröffentlichungsnummer: WO 2002/002086

(56) Entgegenhaltungen:
- EP-A- 0 319 988
- EP-A- 0 582 727
- EP-A- 0 887 075
- WO-A-99/16434
- DE-A- 4 423 850

## Beschreibung

Der Gegenstand der vorliegenden Anmeldung bezieht sich auf ein dermales therapeutisches System mit einer Deckschicht, mit einer Klebermatrix mit einem Gehalt an 2-(3-Benzophenyl)-propionsäure oder [o-(2,6-Dichloranilino)-phenyl]-essigsäure oder einem ihrer in der Pharmazie üblichen Derivate als Wirkstoff und mit einer abziehbaren Schutzschicht, gekennzeichnet durch eine Klebermatrix,
(a) die durch radikalische Copolymerisation von 2-Ethylhexylacrylat, Methylacrylat und Acrylsäure als alleinige Monomere gewinnbar ist oder
(b) bei der das Acrylat-Copolymere aus Einheiten besteht, die ausschließlich von 2-Ethylhexylacrylat, Methylacrylat und Acrylsäure als Monomere herrühren,
wobei die Klebermatrix frei von Penetrationsbeschleunigern ist.

Das System gemäß (a) ist gewinnbar bei einem Verhältnis von Acrylsäure: Methylacrylat von etwa 1:4 und einem Verhältnis von Acrylsäure: 2-Ethylhexylacrylat von 1:9 bis 1:10 (jeweils auf Molbasis oder auf Gewichtsbasis) und das System gemäß (b) ist gekennzeichnet durch von Acrylsäure und Methylacrylat herrührende Einheiten im Verhältnis von etwa 1:4 und durch von Acrylsäure und 2-Ethylhexylacrylat herrührende Einheiten im Verhältnis von 1:9 bis 1:10, jeweils auf Molbasis oder Gewichtsbasis. 2-(3-Benzophenyl)-propionsäure ist bereits 1968 als Substanz patentiert worden. Es hat sich seither bestens in der Therapie von akuten Arthritiden incl. Gichtanfall, chronischen Arthritiden, insbesondere von rheumatoiden Arthritis (chronische Polyarthritis), sowie Spondilitis ankylosans (Morbus Bechterew) und anderen entzündlich-rheumatischen Wirbelsäulenleiden, Reizzuständen bei degenerativen Gelenk- und Wirbelsäulenerkrankungen (Arthrosen und Spondylarthrosen), Weichteilrheumatismus, schmerzhaften Schwellungen oder Entzündungen nach Verletzungen oder Operationen sowie anderen nichtrheumatischen Schmerzzuständen und Dysmenorrhoe bewährt. Da als Nebenwirkungen häufig in dieser Substanzklasse und so auch bei 2-(3-Benzophenyl)-propionsäure Magen- und Darmgeschwüre, Magen-Darm-Beschwerden wie Übelkeit, Erbrechen, Sodbrennen, Magenschmerzen, Völlegefühl, Verstopfung oder Durchfall bei peroraler Applikation auftreten und ein großer Anteil der Indikationen auch topisch einer Behandlung zugänglich ist, insbesondere bei Reizzuständen bei degenerativen Gelenk- und Wirbelsäulenerkrankungen (Arthrosen und Spondylarthrosen), Weichteilrheumatismus, schmerzhaften Schwellungen oder Entzündungen nach Verletzungen und Operationen, wird die Substanz auch in topischen Formulierungen wie Cremes, Salben, Gelen, Sprays etc. angewendet. Dazu penetriert die aus der Formulierung freigesetzte 2-(3-Benzophenyl)-propionsäure die Hautbarriere, um sich im entzündeten Weichteilgewebe aufgrund des entzündungsbedingten sauren Milieus anzureichern und dort zu einem topischen Effekt in der schmerzenden und entzündeten Region des Körperteils zu wirken.
Die Penetration von Arzneistoffen durch die Haut ist weitgehend durch die physikochemischen Eigenschaften der Substanz bestimmt. Hierbei spielen im wesentlichen der Octanol/Wasser-Verteilungskoeffizient sowie die Molekülgröße eine Rolle (Potts RO, Guy RH in: Gurny R, Teubner A; Dermal and transdermal drug delivery, Wiss. Verlagsges. Stuttgart (1993)). Da diese Parameter ohne Molekülmodifikationen nicht beeinflussbar sind, gibt es im wesentlichen nur zwei Möglichkeiten, die Penetrationsrate zu steigern:
1.Erleichterung der Diffusion durch Zusatz von Penetrationsbeschleunigern oder Anwendung von elektrischer Spannung (Iontophorese)
2.Steigerung der Arzneistoffkonzentration in der Grundlage auch über die Löslichkeitsgrenze hinaus (Übersättigung).

Als Penetrationsbeschleuniger werden u.a. Fettsäuren, Fettalkohole, einfache und mehrwertige Alkohole, Laurocapram und Tenside eingesetzt. Viele dieser Substanzen wirken jedoch über eine Störung der Barrierefunktion der Haut und sind damit als mehr oder weniger hautreizend einzustufen. Dennoch sind zahlreiche solche Systeme in Patentschriften beschrieben (vgl. DE 19830649, WO 96229988 etc.).

WO-A-99 16434 offenbart ein den Wirkstoff Diclofenac enthaltendes transdermales therapeutisches System bestehend aus einer Deckschicht, einer wirkstoffhaltigen Matrixschicht aus Durotak® 87-2852 und einer Schutzschicht; ferner ist ein Permeationsbeschleuniger enthalten.

EP-A-0 887 075 offenbart transdermale therapeutische Systeme enthaltend eine Klebermatrix aus Durotak® 87-2852 ohne Zusatz eines Permeationsbeschleunigers für den Wirkstoff Selegilin.

Verträglicher ist die Verwendung von Systemen, bei denen der Wirkstoff in übersättigter Form vorliegt. Üblicherweise ist der maximale Flux einer Substanz durch die Haut durch seine Löslichkeit in der Hornhaut (Stratum corneum), welche die Hauptpenetrationsbarriere darstellt, begrenzt. Diese Sättigungskonzentration wird sich dann einstellen, wenn der Wirkstoff im Vehikel, z.B. in der Matrix des Transdermalsystems, ebenfalls in einer Konzentration vorliegt, die der Löslichkeit im Vehikel entspricht. Eine Möglichkeit, diese sog. maximale thermodynamische Aktivität weiter zu erhöhen, besteht darin, den Arzneistoff in einer die Löslichkeit im Vehikel überschreitenden Konzentration einzuarbeiten. Dies ist z.B. durch die Einarbeitung der 2-(3-Benzophenyl)-propionsäure in Acrylat-Copolymere möglich (DE 19843027). Die Einstellung der Übersättigung muß aber so sensibel erfolgen, dass die Übersättigungen so hoch wie möglich, aber auch so stabil wie nötig sind, da übersättigte Systeme bekanntlich metastabil sind und durch Rekristallisation bei Lagerung in den gesättigten Zustand übergehen. Das hat dann den Nachteil, dass diese Systeme aufgrund der Kristallisation zu Produktreklamationen infolge mangelnden Aspekts als auch mangelnder Klebkraft führen. Ebenfalls ist ein enger Kontakt zwischen dermalen System und der Haut notwendig, um einen wirksamen Anteil an 2-(3-Benzophenyl)-propionsäure in den Zielbereich des entzündeten Weichteilgewebes zu erhalten.

Es wurde nun herausgefunden, dass die Einarbeitung der 2-(3-Benzophenyl)-propionsäure in Form des reinen Enantiomeren oder des Racemats und einer Konzentration von 0,1-30 Gew.%, insbesondere 15-25 Gew.-%, jeweils bezogen auf das Gewicht der Klebermatrix in ein ganz spezielles Acrylat-Copolymer sowohl eine so stabile Übersättigung erreicht, dass ma zu einem wirksamen Produkt kommt, ohne Penetrationsbeschleuniger zusetzen zu müssen als auch eine optimale Haftung auf der Haut erhält, die dergestalt ist, dass bei engen Kontakt zwischen dem dermalen System und der äußeren Hautbarriere über mehrere Tage bis maximal eine Woche trotzdem eine Wiederentfernbarkeit jederzeit gegeben ist, ohne dass es zu Schmerzempfinden noch Hautreizungen kommt. Damit erreicht die Klebkraft des dermalen erfindungsgemäßen Systems eine deutlich längere Tragezeit als beispielsweise im Markt befindliche Produkte, die wasserhaltige Zubereitungen der Art eines Kataplasma oder Breiumschlags enthalten, und wesentlich längeren Kontakt als herkömmliche topische Formulierungen wie Cremes, Gele oder Spray, die durch Kontakt mit Wasser oder Kleidung entfernt werden können.

Es wurden mehrere Acrylat-Copolymere auf Lösungsmittelbasis, wie sie z. B. von der Firma National Starch & Chemical, BV, Zutphen, Netherland unter dem Handelsnamen Durotak zur Verfügung gestellt werden, hinsichtlich ihrer Klebeeigenschaften getestet. Die nachfolgende Tabelle gibt die Copolymer-Zusammensetzung wieder:

| **Monomerzusammensetzung Kleber** | **Durotak 387-2825** | **Durotak 387-2054** | **Durotak 87-2852** | **Durotak 387-2516** | **Durotak 87-2070** |
|---|---|---|---|---|---|
| Butylacrylat | | X | | | |
| Methylmethacrylat | | | | | |
| 2-Ethylhexylacrylat | X | X | X | X | X |
| Methylacrylat | | | X | | |
| Vinylacetat | X | X | | X | X |
| Acrylsäure | X | X | X | | X |
| Glycidyhnetacrylat | | | | X | X |
| 2-Hydroxymethylacrylat | | | | X | |
| Klebeeigenschaften | Rückstände auf der Haut beim Abziehen | Rückstände auf der Haut beim Abziehen | gut | Klebt zu schwach | Klebt zu schwach |

Wie man ersehen kann, werden die Trageeigenschaften nur durch den Einsatz eines Klebers auf Basis von 2-Ethylhexylacrylat, Methylmethacrylat und Acrylsäure erreicht, z.B. durch Durotak 2852, was überraschend ist. Es gibt geringe Modifikationen dieses Klebers von der Firma National Starch & Chemical (Durotak 387-2287, 387-2353), wobei jedoch der oben erwähnte Kleber Durotak 87-2852 zum besten Ergebnis führt. Es handelt sich hierbei um spezielle, unbekannte Wechselwirkungen zwischen Wirkstoff und Acrylat-Copolymer.

Darüber hinaus spielt bei den Trageeigenschaften der Träger bzw. die Deckschicht der Matrix eine wichtige Rolle. Da das dermale System auf Gelenke aufgebracht werden muss, ist eine große Flexibilität erforderlich. Es wurden verschiedene Materialien einem Test unterworfen, der sich über Vliese ("Nonwovens"), Schäume ("Foams"), Folien und Gewebe ("Wovens"). erstreckte. Wichtig war ebenfalls für die Verträglichkeit, dass der eingesetzte Träger eine gute Wasserdampfdurchlässigkeit aufweist. Optimal erwies sich ein längs- und querelastisches Gewebe aus Polyester, welches in weiß oder Hautfarben erhältlich ist (Firma Karl Otto Braun, Germany).

Als Schutzfolie kann eine dem Fachmann bekannte silikonisierte Polyesterfolie, z.B. Hostaphan RN 100 von Diafoil, Hoechst, Germany, easy/easy, eingesetzt werden, die nicht zu dünn sein darf (mind. 36 µm Schichtdicke, vorzugsweise 100 µm Schichtdicke) , damit ein so großes System von 70 bis 140 cm², vorzugsweise von 90 cm² noch gut beim Patienten zu handhaben ist.

Die erfindungsgemäßen dermalen therapeutischen Systeme sind vorzugsweise so beschaffen, dass sie aus einer für den Wirkstoff undurchlässigen Deckschicht, einer auf der Deckschicht haftenden wirkstoffhaltigen Kleberschicht und einer abziehbaren Schutzschicht bestehen.

Diese einfachste Form eines TDS kann in der dem Fachmann wohlbekannten Weise hergestellt werden, dass eine Lösung des Klebers in einem niedrigsiedendem Lösungsmittel mit dem Wirkstoff gemischt wird, die Mischung gleichmäßig auf einer abziehbaren Schutzschicht aufgetragen wird, das Lösungsmittel durch Erwärmen entfernt und das erhaltene Produkt mit einem Träger abgedeckt wird. Die aufgebrachte wirkstoffhaltige Kleberschicht hat eine Dicke von 20 bis 500 µm.

Die nachfolgenden Ausführungsbeispiele dienen zur näheren Erläuterung der Erfindung:

### Beispiel 1:

Zu 30,83 g einer 36 %igen (m/m) Lösung eines Acrylat-Klebstoffes (Durotak 87-2852, National Starch & Chemical B.V., NL-Zutphen) wird eine Lösung von 2,78 g 2-(3-Benzophenyl)-propionsäure in 5,6 g 2-Propanol gegeben. Durch einstündiges Rühren wird die Lösung homogenisiert und anschließend mit einem Rakel auf einer silikonisierten, 100 µm starken Polyesterfolie (FL 2000 100 µ 1-S, Rexam Release B.V., NL-Apeldoorn) in einer Nassschichtdicke von 260 µm ausgestrichen. Nach der Trocknung (1 h bei 40°C und 50 min bei 80°C) wird das klare und homogene Laminat mit einem Polyestergewebe (M02/97, weiß, K.O. Braun, D-Wolfstein) ohne Dehnung kaschiert. Ein Pflaster der Größe 90 cm² enthält bei einem Matrixgewicht von 55,6 g/m² 100 mg 2-(3-Benzophenyl)-propionsäure

### Beispiel 2:

Zu 17,46 g einer 35 %igen (m/m) Lösung eines Acrylat-Klebstoffes (Durotak 87-2852, National Starch & Chemical B.V., NL-Zutphen) wird eine Lösung von 2,08 g 2-(3-Benzophenyl)-propionsäure und 0,21 g Neohesperidin DC in 4,17 g Isopropanol gegeben. Durch einstündiges Rühren wird die Lösung homogenisiert und anschließend mit einem Rakel auf einer silikonisierten, 100 µm starken Polyesterfolie (FL 2000 100 µ 1-S, Rexam Release B.V., NL-Apeldoorn) in einer Nassschichtdicke von 270 µm ausgestrichen. Nach der Trocknung (1 h bei 40°C) wird das leicht trübe Laminat mit einem längs- und querelastischen Polyestergewebe (K. O. Braun, D-Wolfstein) kaschiert. Ein Pflaster der Größe 90 cm² enthält bei einem Matrixgewicht von 55,6 g/m² 125 mg 2-(3-Benzophenyl)-propionsäure .

Wie Tabelle 2 zeigt, weist das Beispiel mit Neohesperidin DC gegenüber den entsprechenden Rezepturen ohne Neohesperidin DC keine veränderte Klebeeigenschaft auf auf.

**Tabelle 2:**

| *Beeinflussung der Klebeeigenschaften von trans- dermalen therapeutischen Systemen durch Neohesperidin DC (Beispiel 4, n=3)* | | |
|---|---|---|
| | 2-(3-Benzophenyl)-propionsäure TDS ohne Zusatz von Neohesperidin DC | 2-(3-Benzophenyl)-propionsäure TDS mit Zusatz von 2,5% Neohesperidin DC (bezogen auf die Matrix) |
| Klebkraft [N/25 mm] | 6,8 ± 0,6 | 6,2 ± 0,4 |
| Trennkraft [N/25 mm] | 0,137 ± 0,012 | 0,127 ± 0,025 |

## Patentansprüche

1. Dermales therapeutisches System mit einer Deckschicht, mit einer Klebermatrix mit einem Gehalt an 2-(3-Benzophenyl)-propionsäure oder [o-(2,6-Dichloranilino)-phenyl]-essigsäure oder einem ihrer in der Pharmazie üblichen Derivate als Wirkstoff und mit einer abziehbaren Schutzschicht, **gekennzeichnet durch** eine Klebermatrix,
(a) die **durch** radikalische Copolymerisation von 2-Ethylhexylacrylat, Methylacrylat und Acrylsäure als alleinige Monomere gewinnbar ist oder
(b) bei der das Acrylat-Copolymere aus Einheiten besteht, die ausschließlich von 2-Ethylhexylacrylat, Methylacrylat und Acrylsäure als Monomere herrühren,
wobei die Klebermatrix frei von Penetrationsbeschleunigern ist.

2. Dermales therapeutisches System nach Anspruch 1, **gekennzeichnet durch** 2-(3-Benzophenyl)-propionsäure in Form des reinen Enantiomeren oder des Racemats.

3. Dermales therapeutisches System nach mindestens einem der vorhergehenden Ansprüche, **gekennzeichnet durch** einen Gehalt an 2-(3-Benzophenyl)-propionsäure einer Konzentration von 0,1 bis 30 Gew.-% und insbesondere 15 bis 25 Gew.-%, jeweils bezogen auf das Gewicht der Klebermatrix mit Wirkstoff.

4. Dermales therapeutisches System nach Anspruch 1 (a), **dadurch gekennzeichnet, daß** es aus Acrylsäure, Methylacrylat und 2-Ethylhexylacrylat bei einem Verhältnis von Acrylsäure: Methylacrylat von etwa 1:4 und einem Verhältnis von Acrylsäure: 2-Ethylhexylacrylat von 1:9 bis 1:10 (jeweils auf Molbasis oder auf Gewichtsbasis) gewinnbar ist.

5. Dermales therapeutisches System nach Anspruch 1 (b), **gekennzeichnet durch** von Acrylsäure und Methylacrylat herrührende Einheiten im Verhältnis von etwa 1:4 und **durch** von Acrylsäure und 2-Ethylhexylacrylat herrührende Einheiten im Verhältnis von 1:9 bis 1:10, jeweils auf Molbasis oder Gewichtsbasis.

6. Dermales therapeutisches System nach mindestens einem der vorhergehenden Ansprüche, **gekennzeichnet durch** eine Schichtdicke der Klebermatrix von 20 bis 500 µm.

7. Dermales therapeutisches System nach mindestens einem der vorhergehenden Ansprüche, **dadurch gekennzeichnet, dass** es keinen Permeationsbeschleuniger enthält.

8. Dermales therapeutisches System nach mindestens einem der vorhergehenden Ansprüche, **gekennzeichnet durch** ein längsund querelastisches Gewebe als Deckschicht.

9. Dermales therapeutisches System nach Anspruch 8, **gekennzeichnet durch** ein Polyestergewebe als Deckschicht.

10. Dermales therapeutisches System nach Anspruch 8 und/oder 9, **gekennzeichnet durch** ein Polyestergewebe aus Kettund Schußfäden von Polybutylentherephthalat oder Polyethylentherephthalat.

## Claims

1. Dermal therapeutic system with a covering layer, with an adhesive matrix containing 2-(3-benzophenyl)propionic acid or [o-(2,6-dichloroanilino)phenyl]acetic acid or one of their derivatives conventionally used in pharmacy, as the active ingredient, and with a pull-off protective layer, **characterized by** an adhesive matrix
(a) which is obtainable by the free-radical copolymerization of 2-ethylhexyl acrylate, methyl acrylate and acrylic acid as the only monomers, or
(b) in which the acrylate copolymer consists of units originating exclusively from 2-ethylhexyl acrylate, methyl acrylate and acrylic acid as monomers, the adhesive matrix being free of penetration accelerators.

2. Dermal therapeutic system according to Claim 1, **characterized by** 2-(3-benzophenyl)propionic acid in the form of a pure enantiomer or the racemate.

3. Dermal therapeutic system according to at least one of the preceding claims, **characterized in that** it contains 2-(3-benzophenyl)propionic acid in a concentration of 0.1 to 30 wt.% and especially of 15 to 25 wt.%, based in each case on the weight of the adhesive matrix containing the active ingredient.

4. Dermal therapeutic system according to Claim 1 (a); **characterized in that** it is obtainable from acrylic acid, methyl acrylate and 2-ethylhexyl acrylate, the acrylic acid/methyl acrylate ratio being about 1:4 and the acrylic acid/2-ethylhexyl acrylate ratio being 1:9 to 1:10 (on a molar or weight basis in each case).

5. Dermal therapeutic system according to Claim 1 (b), **characterized by** units originating from acrylic acid and methyl acrylate in a ratio of about 1:4 and by units originating from acrylic acid and 2-ethylhexyl acrylate in a ratio of 1:9 to 1:10, on a molar or weight basis in each case.

6. Dermal therapeutic system according to at least one of the preceding claims, **characterized in that** the adhesive matrix has a layer thickness of 20 to 500 µm.

7. Dermal therapeutic system according to at least one of the preceding claims, **characterized in that** it does not contain a permeation accelerator.

8. Dermal therapeutic system according to at least one of the preceding claims, **characterized in that** the covering layer is a fabric with longitudinal and transverse elasticity.

9. Dermal therapeutic system according to Claim 8, **characterized in that** the covering layer is a polyester fabric.

10. Dermal therapeutic system according to Claim 8 and/or 9, **characterized in that** the polyester fabric is composed of warp and weft threads of polybutylene terephthalate or polyethylene terephthalate.

## Revendications

1. Système thérapeutique dermique, doté d'une couche de recouvrement, d'une matrice d'adhésif qui contient comme produit actif une proportion d'acide 2-(3-benzophényl)propionique, de l'acide [o-(2,6-dichloroanilino)-phényl]acétique ou de l'un de leurs dérivés habituels en pharmacie, et une couche de protection apte à être enlevée, **caractérisé par** une matrice d'adhésif :
qui peut être obtenue par copolymérisation radicalaire d'acrylate de 2-éthylhexyle, d'acrylate de méthyle et d'acide acrylique comme uniques monomères ou
dans laquelle le copolymère d'acrylates est constitué d'unités exclusivement basées sur les monomères d'acrylate de 2-éthylhexyle, d'acrylate de méthyle et d'acide acrylique
la matrice d'adhésif étant exempte d'accélérateurs de pénétration.

2. Système thérapeutique dermique selon la revendication 1, **caractérisé en ce que** l'acide 2-(3-benzophényl)propionique se présente sous la forme de l'énantiomère pur ou de son racémate.

3. Système thérapeutique dermique selon au moins l'une des revendications précédentes, **caractérisé en ce que** la teneur en acide 2-(3-benzophényl)propionique qui correspond à une concentration comprise entre 0,1 et 30 % en poids et en particulier de 15 à 25 % en poids par rapport à la matrice d'adhésif avec produit actif.

4. Système thérapeutique dermique selon la revendication 1 (a), **caractérisé en ce qu'**il peut être obtenu à partir d'acide acrylique, d'acrylate de méthyle et d'acrylate de 2-éthylhexyle dans une proportion entre l'acide acrylique et l'acrylate de méthyle d'environ 1:4 et dans une proportion entre l'acide acrylique et l'acrylate de 2-éthylhexyle de 1:9 à 1:10, chaque fois sur base molaire ou sur base pondérale.

5. Système thérapeutique dermique selon la revendication 1 (b), **caractérisé par** des unités à base d'acide acrylique et d'acrylate de méthyle dans une proportion entre l'acide acrylique et l'acrylate de méthyle d'environ 1:4 et par des unités à base d'acide acrylique et d'acrylate de 2-éthylhexyle dans une proportion de 1:9 à 1:10, chaque fois sur base molaire ou sur base pondérale.

6. Système thérapeutique dermique selon au moins l'une des revendications précédentes, **caractérisé par** une couche de matrice d'adhésif d'une épaisseur de 20 à 500 µm.

7. Système thérapeutique dermique selon au moins l'une des revendications précédentes, **caractérisé en ce qu'**il ne contient pas d'accélérateur de perméation.

8. Système thérapeutique dermique selon au moins l'une des revendications précédentes, **caractérisé par** un tissu élastique dans le sens longitudinal et dans le sens transversal comme couche de recouvrement.

9. Système thérapeutique dermique selon la revendication 8, **caractérisé par** un tissu de polyester comme couche de recouvrement.

10. Système thérapeutique dermique selon les revendications 8 et/ou 9, **caractérisé par** un tissu de polyester dont les fils de chaîne et les fils de trame sont en poly(téréphtalate de butylène) ou en poly(téréphtalate d'éthylène).
